# EUROPEAN PATENT APPLICATION

(11) **EP 2 100 872 A1**
(43) Date of publication of application: **16.09.2009**
(21) Application number: 07807648.6
(22) Date of filing: 20.09.2007
(51) Int. Cl.: C07C 51/235, C07C 57/055, C08F 22/06

(54) **ACRYLIC ACID PRODUCTION PROCESS, ACRYLIC ACID PRODUCTION APPARATUS, AND COMPOSITION FOR PRODUCTION OF ACRYLIC ACID**

(30) Priority: 02.11.2006 JP 2006299462
(71) Applicant: Nippon Shokubai Co., Ltd., Osaka-shi, Osaka 541-0043 (JP)
(72) Inventor: KASUGA, Hiroto, Himeji-shi, Hyogo 671-1154 (JP); MATSUNAMI, Etsushige, Himeji-shi, Hyogo 671-1242 (JP); TAKAHASHI, Tsukasa, Himeji-shi, Hyogo 671-1242 (JP)
(74) Representative: Henkel, Feiler & Hänzel
(86) International application number: PCT/JP2007/068296
(87) International publication number: WO 2008/053646

(57) **Abstract**

There are provided a process, an apparatus, and an acrolein-containing composition, for producing acrylic acid from the acrolein-containing composition at a high yield. The process for producing acrylic acid includes a refinement step of removing phenol and/or 1-hydroxyacetone from an acrolein-containing composition and an oxidation step of oxidizing acrolein in the acrolein-containing composition after the refinement step to produce acrylic acid, and the apparatus to be used in the process includes a refiner to be used in the refinement step and an oxidation reactor for oxidizing acrolein to produce acrylic acid. The acrolein-containing composition is a composition having a (mass of phenol) / (mass of acrolein) ratio of 0.020 or lower and a (mass of 1-hydroxyacetone) / (mass of acrolein) ratio of 0.020 or lower.

## Description

### TECHNICAL FIELD

The present invention relates to a process for producing acrylic acid, an apparatus for producing acrylic acid, and a composition for producing acrylic acid.

### BACKGROUND ART

Acrolein has been used as a raw material of acrolein derivatives such as 1,3-propanediol, methionine, acrylic acid, and 3-methylthiopropionaldehyde. It has been known that acrolein can be produced by dehydration of glycerin.

For example, Japanese Patent Laid-open Publication (Kokai) No. Hei 6-211724 (U.S. Patent No. 5,387,720) discloses that an acrolein-containing composition containing allyl alcohol, acetaldehyde, propionaldehyde, and 1-hydroxyacetone as by-products can be produced by dehydration of glycerin and further discloses a process for producing 1,3-propanediol by reduction of acrolein in a raw material which is obtained by refinement of the above composition through distillation to increase its acrolein concentration. In addition, Japanese Patent Laid-open Publication (Kokai) No. 2005-213225 (U.S. Patent Application Publication Serial No. 2007/0129570) discloses a process for producing acrylic acid by gas-phase oxidation of acrolein in a composition which is produced by gas-phase dehydration of glycerin.

As described above, various compounds are produced as acrolein derivatives from acrolein, and even when any of the derivatives is produced, it is desired to produce it at a high yield. A production process with a high yield is also desired without exceptions even when acrylic acid is produced.

### DISCLOSURE OF THE INVENTION

In view of the above circumstances, it is an object of the present invention to provide a technique of producing acrylic acid from an acrolein-containing composition at a high yield.

The present inventors have made various studies on whether some compounds contained in an acrolein-containing composition affect the yield of acrylic acid. As a result, they have found that phenol and 1-hydroxyacetone cause a decrease in the yield of acrylic acid, which has led to the completion of the present invention.

That is, the present invention provides a process for producing acrylic acid, comprising a refinement step of removing phenol and/or 1-hydroxyacetone from an acrolein-containing composition and an oxidation step of oxidizing acrolein in the acrolein-containing composition after the refinement step to produce acrylic acid. In this regard, the "acrolein-containing composition" as used in the production process of the present invention may be a composition containing acrolein and phenol; a composition containing acrolein and 1-hydroxyacetone; or a composition containing acrolein, phenol, and 1-hydroxyacetone; and it may be a composition in the form of a liquid or gas.

The above process for producing acrylic acid may comprise a dehydration step of dehydrating glycerin to produce acrolein before the refinement step. This dehydration step is, for example, a step of dehydrating glycerin in the gas phase.

The present invention further provides an apparatus for producing acrylic acid, comprising a refiner for removing phenol and/or 1-hydroxyacetone from an acrolein-containing composition and an oxidation reactor for oxidizing acrolein in the acrolein-containing composition refined by the refiner to produce acrylic acid.

The present invention further provides a process for producing an acrylic acid derivative, comprising a step of producing acrylic acid by use of the above process for producing acrylic acid. In this regard, the "acrylic acid derivative" is any of the compounds produced using acrylic acid as a raw material, and examples thereof may include acrylic acid esters, polyacrylic acids, polyacrylates, and water-absorbing resins.

The present invention further provides a composition for producing acrylic acid, comprising acrolein, wherein a ratio (P/A) of a mass (A) of acrolein and a mass (P) of phenol is 0.020 or lower and a ratio (H/A) of a mass (A) of acrolein and a mass (H) of 1-hydroxyacetone is 0.020 or lower.

According to the production process and apparatus of the present invention, phenol and/or 1-hydroxyacetone are/is removed from an acrolein-containing composition, and therefore, acrylic acid can be produced at a high yield.

Further, according to the composition of the present invention, the amount(s) of phenol and/or 1-hydroxyacetone to the amount of acrolein are/is equal to or lower than the prescribed amount(s), and therefore, acrylic acid can be produced at a high yield.

### BRIEF DESCRIPTION OF THE DRAWINGS

[Fig. 1] This is a schematic configuration diagram for explaining one example of a refiner in an embodiment of the present invention.
[Fig. 2] This is a schematic configuration diagram for explaining another example of a refiner in an embodiment of the present invention.

### BEST MODE FOR CARRYING OUT THE INVENTION

The present invention will be described below based on an embodiment. The process for producing acrylic acid in this embodiment is a process comprising a refinement step of removing phenol and/or 1-hydroxyacetone from an acrolein-containing composition and an oxidation step of oxidizing acrylic acid after the refinement step. The process in this embodiment will be described below step by step.

First, the refinement step will be described.

The acrolein-containing composition to be used in the refinement step contains phenol and/or 1-hydroxyacetone. The acrolein-containing composition is produced by gas-phase dehydration of glycerin using glycerin gas or by liquid-phase dehydration of glycerin using glycerin in the form of a liquid. In these dehydrations, a catalyst is used.

The catalysts which can be used in the dehydration may be those having solid acidity, and examples thereof may include natural or synthetic clay compounds, such as zeolites represented by H-ZSMS, kaolinites, bentonites, and montmorillonites; catalysts obtained by allowing sulfuric acid, phosphoric acid, or phosphates (e.g., alkali metal salts of phosphoric acid, manganese phosphate, zirconium phosphate) to be supported on alumina or others; inorganic oxides or inorganic composite oxides, such as Al_{2O3}, TiO₂, ZrO₂, SnO₂, V₂O₅, SiO₂-Al₂O₃, SiO₂-TiO₂, and TiO₂-WO₃; solid acidic substances such as sulfates, carbonates, nitrates, and phosphates of metals, e.g., MgSO₄ Al₂(SO₄)₃, K₂SO₄, AlPO₄, Zr₃(SO₄)₂. In addition, zirconium oxide supporting phosphoric acid, sulfuric acid, or tungsten oxide, and solid acids disclosed in the International Publications WO 2006/087083 and WO 2006/087084 can be used as a catalyst for dehydration of glycerin. The shape of these catalysts to be used for dehydration is not limited, but they may preferably have a spherical, column-like, ring-like, or saddle-like shape.

The production conditions for an acrolein-containing composition will be described in more detail by reference to the gas-phase dehydration of glycerin as an example. The gas-phase dehydration is carried out by bringing a reaction gas containing glycerin into contact with a catalyst in a reactor arbitrarily selected from fixed-bed reactors, fluidized-bed reactors, and others.

The reaction gas may be a gas composed only of glycerin or one containing a gas for adjusting the glycerin concentration in the reaction gas. Examples of the gas for concentration adjustment may be water vapor, nitrogen, and air. Further, the glycerin concentration in the reaction gas may be from 0.1 to 100 mol%, preferably 1 mol% or higher, and it may be 10 mol% or higher for the purpose of producing acrolein with high efficiency from an economical point of view.

In the gas-phase dehydration, a reaction gas may preferably be allowed to pass through a reactor in terms of production efficiency, and the flow rate of the reaction gas in this case may be from 100 to 10,000 hr⁻¹ when expressed by reaction gas flow rate per unit catalyst volume (GHSV). It may preferably be 5,000 hr⁻¹ or lower, and it may be 3,000 hr⁻¹ or lower for the purpose of producing acrolein with high efficiency from an economical point of view. Further, the gas-phase dehydration temperature is not particularly limited, but it may be from 200°C to 500°C, preferably from 250°C to 450°C, and more preferably from 300°C to 400°C, and the pressure of the reaction gas may be a pressure in such a range that glycerin gas is not condensed, usually from 0.001 to 1 MPa, and preferably from 0.01 to 0.5 MPa.

According to the gas-phase dehydration of glycerin, an acrolein-containing composition in the form of a gas is produced. For the purpose of collecting the composition in the form of a gas, there may be used a method of allowing the acrolein-containing composition in the form of a gas to be cooled to become a liquid or a method of allowing the acrolein-containing composition in the form of a gas to be absorbed in a solvent having acrolein dissolving ability, such as water.

Incidentally, as described above, the present inventors have found, based on the results of researches, that an acrolein-containing composition from which phenol or 1-hydroxyacetone has been removed may be preferred as a composition for producing acrylic acid. If the background to this finding is described, it is as follows.

The present inventors have made an experiment on the production of acrylic acid, in which to acrolein is added phenol, 1-hydroxyacetone, or allyl alcohol, all of which are produced as by-products in the production of acrolein by dehydration of glycerin, and the resultant composition is used as a raw material.

In this connection, the above experiment was made as follows. A reaction gas was allowed to pass (GHSV: 2,000 hr⁻¹) through a stainless steel reaction tube filled with 20 cc of a catalyst for producing acrylic acid (the catalyst for producing acrylic acid in Example 1 described below), and acrylic acid was produced at a reaction temperature of 230°C. The gas flowing out of the reaction tube was collected in a period of from 60 to 80 minutes from the start of flowing, and components contained therein were analyzed by gas chromatography. The reaction gas used in this experiment was a gas containing, in addition to 54.4 parts by volume of nitrogen, 10.44 parts by volume of oxygen, and 1.8 parts by volume of acrolein, 33.36 parts by volume of water vapor (Experiment No.1); 33.18 parts by volume of water vapor and 0.18 parts by volume of phenol (Experiment No. 2); 33.00 parts by volume of water vapor and 0.36 parts by volume of 1-hydroxyacetone (Experiment No.3); or 33.00 parts by volume of water vapor and 0.36 parts by volume of allyl alcohol (Experiment No. 4).

The results of this experiment are shown in the following Table 1.

**[Table 1]**

| Experiment No. | Additive | Additive amount (parts by volume) | Acr conversion rate (%) | Yield (%) | |
|---|---|---|---|---|---|
| | | | | AA | AcOH |
| 1 | None | - | 91.9 | 81.7 | 3.6 |
| 2 | PhOH | 0.18 | 58.8 | 45.2 | 0.2 |
| 3 | HO-Act | 0.36 | 86.8 | 76.1 | 19.0 |
| 4 | AlOH | 0.36 | 86.9 | 86.8 | 0.5 |

| | | | | | |
|---|---|---|---|---|---|
| Acr: acrolein, AA: acrylic acid, AcOH: acetic acid, HO-Act: 1-hydroxyacetone, PhOH: phenol, AlOH: allyl alcohol Additive amount: ratio to 1.8 parts by volume of acrolein Conversion rate, Yield: calculated on the basis of the number of carbon atoms of acrolein | | | | | |

As shown in Table 1, when an acrolein-containing composition containing phenol (Experiment No. 2) or 1-hydroxyacetone (Experiment No. 3) was used as a raw material, the conversion rate of acrolein and the yield of acrylic acid were lower than those obtained when an acrolein composition containing none of these compounds was used as a raw material. From this reason, it was decided to remove phenol or 1-hydroxyacetone from an acrolein-containing composition.

From the results of the above experiment, it has been found that the production of acetic acid as a by-product can be suppressed when 1-hydroxyacetone is removed.

As described above, when the acrolein-containing composition contains phenol or 1-hydroxyacetone, the yield of acrylic acid is decreased, and it is supposedly attributed to the following reason. Similarly to the fact that phenol and acrolein are polymerized, if an acid or a base exists, to become a polymer, it is supposed that a polymerized compound could be produced at the acid or base points of a catalyst for producing acrylic acid. Then, as a result of the production of the above compound even at the acid or other points of a catalyst for producing acrylic acid, it is supposed that the active points of the catalyst are covered to decrease the yield of acrylic acid. Further, when the acrolein-containing composition contains 1-hydroxyacetone, because a great amount of acetic acid is produced, it is presumed that 1-hydroxyacetone subjected to the oxidation is converted into acetic acid. Such conversion is supposed to decrease the catalytic active points for converting acrolein into acrylic acid to decrease the yield of acrylic acid.

Phenol and 1-hydroxyacetone are compounds which lower the yield of acrylic acid, and therefore, the removal amounts of both compounds are more preferred as these amounts are greater. With respect to the amount of phenol in the acrolein-containing composition after removal, when expressed by a ratio (P/A) of a mass of acrolein (A) and a mass of phenol (P), P/A may preferably be 0.020 or lower, more preferably 0.010 or lower, and still more preferably 0.005 or lower. Further, with respect to the amount of 1-hydroxyacetone in the acrolein-containing composition after removal, when expressed by a ratio (H/A) of a mass of acrolein (A) and a mass of 1-hydroxyacetone (H), H/A may preferably be 0.020 or lower, more preferably 0.010 or lower, and still more preferably 0.005 or lower.

As described above, the removal amounts of both compounds are more preferred as these amounts are greater; however, a problem of an increase in the loss amount of acrolein and a problem of complication of the refinement step occur accompanying an increase in the removal amounts. These problems tend to be caused when the removal of phenol is carried out by a refining method involving heating, such as distillation operation. Taking this fact into consideration, P/A may be 1 x 10⁻⁹ or higher, preferably 1 x 10⁻⁷ or higher, and more preferably 1 x 10⁻⁵ or higher. Further, even in the removal of 1-hydroxyacetone, when the above problems are caused, H/A may be 1 x 10⁻⁹ or higher, preferably 1 x 10⁻⁷ or higher, and more preferably 1 x 10⁻⁵ or higher.

The refinement step is to be carried out so as to adjust the H/A and P/A of an acrolein-containing composition within the above ranges. In this refinement step, there may be used a refiner appropriately selected from the heretofore known refiners. In the refinement step, the separation of water or other impurities in the acrolein-containing composition by solvent extraction may be carried out, if necessary, before the refinement of the acrolein-containing composition using a refiner.

Examples of the refiner to be used in the refinement step of this embodiment may include refiners provided with a distillation tower for fractionating acrolein having a low boiling point in an acrolein-containing composition in the form of a liquid; a condensation tower for condensing and separating phenol and/or 1-hdyroxyacetone having a high condensation temperature in an acrolein-containing composition in the form of a gas; and an evaporation tower for evaporating acrolein having a low boiling point by blowing a gas to a stored acrolein-containing composition in the form of a liquid. The exemplified refiners utilize the boiling point difference of acrolein (having a boiling point of 53°C), phenol (having a boiling point of 182°C), and 1-hydroxyacetone (having a boiling point of 146°C) and can easily remove phenol and/or 1-hydroxyacetone.

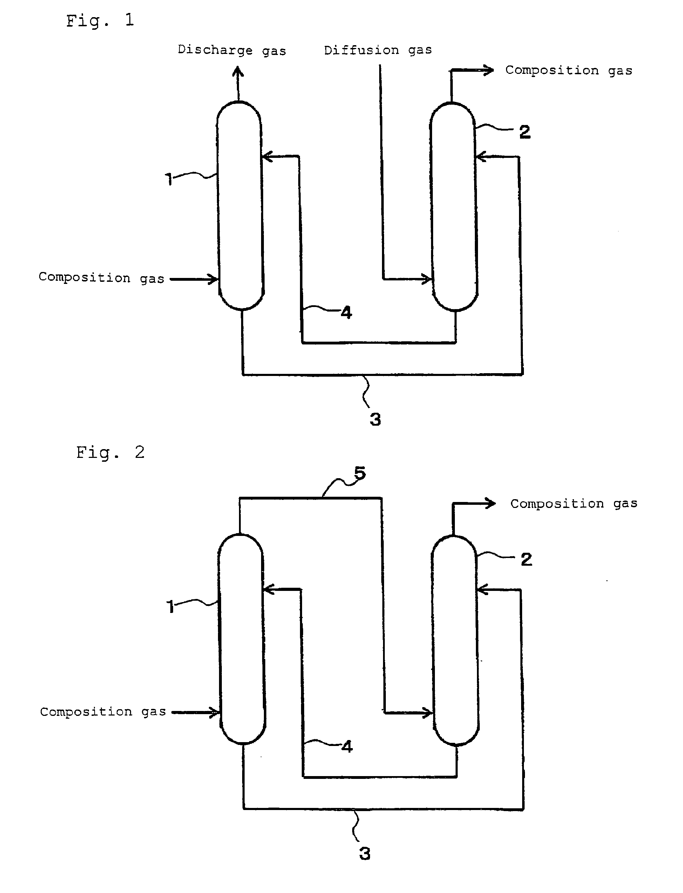

Fig. 1 is a schematic configuration diagram for explaining one example of a refiner in an embodiment of the present invention. A refiner provided with a diffusion tower exemplified above will further be described by reference to Fig. 1. The refiner shown in this figure is composed of a collection tower 1 for allowing an acrolein-containing composition in the form of a gas to be condensed to become a liquid by cooling and a diffusion tower 2 for evaporating acrolein in an acrolein-containing composition in the form of a liquid. Of these towers, in the collection tower 1, an acrolein-containing composition in the form of a gas (the "composition gas" in Fig. 1) is supplied from the tower bottom portion thereof and a discharge gas which has not been condensed to become a liquid by cooling in the collection tower 1 is released from the tower top portion. On the other hand, in the diffusion tower 2, a diffusion gas for evaporating the acrolein-containing composition in the form of a liquid is supplied from the tower bottom portion thereof and an acrolein-containing composition gas in the form of a gas is released from the tower top portion. Then, the collection tower 1 and the diffusion tower 2 are connected with pipes as follows. The tower bottom portion of the collection tower 1 and the tower top portion of the diffusion tower 2 are connected with a delivery pipe 3 and the tower bottom portion of the diffusion tower 2 and the tower top portion of the collection tower 1 are connected with a return pipe 4.

The refinement step of an acrolein-containing composition in a refiner with the above configuration shown in Fig. 1 will be described. An acrolein-containing composition in the form of a liquid, which has become a liquid by cooling in the collection tower 1, is supplied to the diffusion tower 2 through the delivery pipe 3. The acrolein-containing composition in the form of a liquid is stored at a prescribed amount in the diffusion tower 2 and the acrolein-containing composition in the form of a liquid exceeding the prescribed amount due to excess supply is turned back to the collection tower 1 through the return pipe 4. On the other hand, the diffusion gas is supplied to the acrolein-containing composition in the form of a liquid, which has been stored in the diffusion tower 2, and therefore, acrolein having a lower boiling point than those of phenol and 1-hydroxyacetone is preferentially evaporated and a gas containing the evaporated acrolein as a composition gas is released out of the tower top portion of the diffusion tower 2. In addition, the released composition gas may be introduced directly to the oxidization reactor described below.

As described above, the diffusion gas is to be supplied to the diffusion tower 2. The diffusion gas may be at a temperature to an extent that acrolein can be evaporated. Then, as the diffusion gas, air, nitrogen, and water vapor may be used, and an economically preferable gas discharged out of the acrylic acid absorption tower as described below may also be used. Further, as the economically preferable diffusion gas, the discharge gas from the collection tower 1 can also be used. Fig. 2 is a schematic configuration diagram of a refiner in which the discharge gas from the collection tower 1 is used as the diffusion gas, and this refiner is obtained by connecting the tower top portion of the collection tower 1 and the tower bottom portion of the diffusion tower 2 in the refiner shown in Fig. 1 with a gas introduction pipe 5.
The use of a refiner with the configuration shown in Fig. 2 makes it possible to use the discharge gas from the collection tower 1 as the diffusion gas, and in addition, acrolein in the discharge gas which cannot be collected in the collection tower 1 can entirely be supplied to the diffusion tower 2.

Then, the oxidation step will be described.

In the oxidation step in this embodiment, a catalyst and a gas of the acrolein-containing composition subjected to the refinement step are allowed to coexist in an oxidation reactor arbitrarily selected from fixed-bed reactors, fluidized-bed reactors, or others, so that acrolein is oxidized at 200°C to 400°C in gas phase to produce acrylic acid.

The catalyst to be used in the above oxidation is not particularly limited so long as it is a catalyst to be used when acrylic acid is produced by a gas-phase catalytic oxidation method using acrolein or an acrolein-containing gas and molecular oxygen or a molecular oxygen-containing gas. Examples thereof may include mixtures of metal oxides such as iron oxide, molybdenum oxide, titanium oxide, vanadium oxide, tungsten oxide, antimony oxide, tin oxide, and copper oxide; and composites of metal oxides. Of these exemplified catalysts, molybdenum-vanadium type catalysts containing molybdenum and vanadium as the main constituent metals may be preferred. Further, the catalyst may also be that obtained by allowing the above mixture and/or composite to be supported on a carrier (e.g., zirconia, silica, alumina, and composites thereof, and silicon carbide) .

The gas of an acrolein-containing composition to be used in the oxidation step may be preferred to contain oxygen in terms of oxidation promotion; however, when the amount of oxygen added is too excess, combustion may occur and it may be accompanied with a risk of explosion, and therefore, the upper limit thereof should appropriately be set.

Acrylic acid is produced by the above oxidation step; however, acrylic acid produced in the gas-phase oxidation step becomes acrylic acid in the form of a gas. In order to recover the acrylic acid, an absorption tower is used, in which the acrylic acid can be cooled or absorbed in a solvent such as water.

The produced acrylic acid has heretofore been known to be usable as a raw material for acrylic acid derivatives such as acrylic acid esters, polyacrylic acids, or others, and therefore, the above process for producing acrylic acid can be served as a step of producing acrylic acid in a process for producing an acrylic acid derivative.

Then, when polyacrylic acid is produced using the acrylic acid obtained, an aqueous solution polymerization method or a reverse-phase suspension polymerization method may be employed to produce polyacrylic acid which can be used as a water-absorbing resin. In this regard, the aqueous solution polymerization method is a method in which acrylic acid in an aqueous acrylic acid solution is polymerized without using a dispersion solvent, and this method is disclosed in the U.S. Patents Nos. 4,625,001, 4,873,299, 4,286,082, 4,973,632, 4,985,518, 5,124,416, 5,250,640, 5,264,495, 5,145,906, and 5,380,808, and the E.P. Patents Nos. 0 811 636, 0 955 086, and 0 922 717. Further, the reverse-phase suspension method is a polymerization method in which an aqueous solution of acrylic acid as a monomer is suspended in a hydrophobic organic solvent, and this method is disclosed in the U.S. Patents Nos. 4,093,776, 4,367,323, 4,446,261, 4,683,274, and 5,244,735.

### EXAMPLES

The present invention will be described below in more detail by way of Examples, but the present invention is not limited to the following Examples. The present invention can be put into practice after appropriate modifications or variations within a range meeting the gists described above and later, all of which are included in the technical scope of the present invention.

An acrolein-containing composition was prepared using a catalyst for producing acrolein and the prepared composition was refined. Then, the composition after the refining was used as a raw material to produce acrylic acid. The details of this production process are as follows.

### Catalyst for producing acrolein

First, 0.4387 g of Cs₂CO₃ and 3.0629 g of ammonium dihydrogenphosphate were dissolved in 350 g of water, with which 40 g of SiO₂ powder was impregnated at normal temperature, and the mixture was evaporated to dryness to obtain a catalyst precursor. Then, the catalyst precursor was baked under conditions at 600°C in the air for 5 hours, and the resultant product was pulverized and classified to prepare a catalyst for producing acrolein, having a particle diameter of from 0.7 to 2.0 mm.

### Preparation of acrolein-containing composition

A stainless steel reaction tube (having an inner diameter of 10 mm and a length of 500 mm) filled with 15 ml of the above catalyst for producing acrolein was provided as a fixed-bed reactor, and the reactor was immersed in a salt bath at 360°C. Then, nitrogen gas was allowed to pass through the reactor at a flow rate of 61.5 ml/min for 30 minutes, and a reaction gas (the reaction gas composition thereof was 27 mol% glycerin, 34 mol% water, and 39 mol% nitrogen gas) was allowed to pass through the reactor at a flow rate of 632 hr⁻¹. The gas flowing out of the reactor was cooled to become a liquid, which was collected to prepare an acrolein-containing composition. The amount of liquid collected was 94% by mass of the aqueous glycerin solution used.

The prepared acrolein-containing composition was quantitatively analyzed by gas chromatography (GC-14B (the detector thereof was FID), available from Shimadzu Corporation, using Packed Column ZT-7, available from Shinwa Chemical Industries Ltd.), and as a result, the following analysis values were obtained: 31.0% by mass of acrolein, 1.3% by mass of phenol, 7.5 % by mass of 1-hydroxyacetone, 0.1% by mass of glycerin, and 0.1% by mass of allyl alcohol. Further, as a result of the analysis by the Karl-Fischer method, an analysis value of 54% by mass of water was obtained.

### Refining of acrolein-containing composition

Using a packed tower filled with Dickson packings, having a theoretical plate number equivalent to 5 plates, the above acrolein-containing composition was refined by distillation under conditions at a tower top temperature of 46°C, at a tower bottom temperature of 100°C, at a reflux ratio of 3.3, and at normal pressure.

The refined acrolein-containing composition was quantitatively analyzed by gas chromatography, and as a result, the following analysis results were obtained: 96.7% by mass of acrolein and 0.05% by mass of phenol, and 1-hydroxyacetone, glycerin, and allyl alcohol were not detected.

### Production of acrylic acid

A stainless steel reaction tube (having an inner diameter of 25 mm and a length of 500 mm) filled with 20 ml of a catalyst for producing acrylic acid was provided as a fixed-bed oxidization reactor, and the reactor was placed in a nitrate bath at 230°C. Then, an acrolein-containing gas was allowed to pass through the reactor. In this regard, as the acrolein-containing gas, there was used a mixed gas containing a mixture of 1 part by mass of the refined acrolein-containing composition and 5.75 parts by mass of water (at a flow rate of 0.209 g/min), air (at a flow rate of 331.4 ml/min), and nitrogen gas (at a flow rate of 31.2 ml/min). After 24 hours from the start of the passing of the acrolein gas, the gas flowing out of the reactor was cooled to become a liquid, which was collected to obtain an acrylic acid-containing composition.

The above catalyst for producing acrylic acid was prepared as follows. First, 350 g of ammonium paramolybdate, 116 g of ammonium metavanadate, and 44.6 g of ammonium paratungstate were dissolved in 2,500 ml of water heated under stirring, to which 1.5 g of vanadium trioxide was added. Separately, 87.8 g of copper nitrate was dissolved in 750 ml of water heated under stirring, to which 1.2 g of cuprous oxide and 29 g of antimony trioxide were added. These two solutions were mixed together, to which 1,000 ml of spherical α-alumina having a diameter of from 3 to 5 mm as a carrier was added, and the resultant mixture was evaporated to dryness under stirring to obtain a catalyst precursor. The catalyst precursor was baked at 400°C for 6 hours to prepare a catalyst for producing acrylic acid. The supporting metal composition of the catalyst for producing acrylic acid was Mo₁₂V_{6.1}W₁Cu_{2.3}Sb_{1.2}.

Further, the production of acrylic acid (Comparative Example) was carried out as a comparison with the above Example. In this Comparative Example, the production of acrylic acid was carried out in the same manner as described in Example, except that: (1) an un-refined acrolein-containing composition which has not been distilled was used; and (2) as the acrolein-containing gas to be allowed to pass through the reactor in the production of acrylic acid, a mixed gas containing a mixture of 1 part by mass of the un-refined acrolein-containing composition and 1.3 parts by mass of water (at a flow rate of 0.209 g/min), air (at a flow rate of 331.4 ml/min), and nitrogen gas (at a flow rate of 31.2 ml/min) was used.

Acrolein and acrylic acid contained in the acrylic acid-containing compositions produced in the above Example and Comparative Example were quantitatively analyzed by gas chromatography to calculate the conversion rate of acrolein, the yield of acrylic acid, and the selectivity of acrylic acid. The calculation results are shown in Table 2. In Table 2, both of the yield and selectivity were values calculated on the basis of acrolein in the reaction gas.

**[Table 2]**

| | Reaction gas (g/min) | | | Reaction gas composition mass ratio | | Acr conversion rate (%) | Yield (%) | | Selectivity (%) | |
|---|---|---|---|---|---|---|---|---|---|---|
| | Acr | PhOH | HO-Act | P/A | H/A | | AA | AcOH | AA | AcOH |
| Example | 0.030 | 0.00002 | 0.00000 | 0.0007 | - | 91.0 | 80.9 | 3.7 | 88.9 | 4.0 |
| Comp. Ex. | 0.030 | 0.00126 | 0.00727 | 0.0420 | 0.2423 | 64.3 | 33.1 | 26.5 | 51.5 | 41.2 |

| | | | | | | | | | | |
|---|---|---|---|---|---|---|---|---|---|---|
| Acr: acrolein, AA: acrylic acid, AcOH: acetic acid, HO-Act: 1-hydroxyacetone, PhOH: phenol A: Mass of acrolein in reaction gas P: Mass of phenol in reaction gas H: Mass of 1-Hydroxyacetone in reaction gas Conversion rate, Yield, Selectivity: Calculated on the basis of the number of carbon atoms in acrolein | | | | | | | | | | |

As shown in Table 2, Example in which there was used an acrolein composition from which phenol and 1-hydroxyacetone had been removed was excellent in all of the conversion rate of acrolein, the yield of acrylic acid, and the selectivity of acrylic acid than Comparative Example. Further, it can be confirmed that the yield and selectivity of acetic acid as a byproduct were suppressed to be low in Example in which 1-hydroxyacetone was removed.

A water-absorbing resin was produced using, as a raw material, acrylic acid produced by a method according to the above Example. The production process and physical properties of the water-absorbing resin are as follows.

### Production of water-absorbing resin

An acrolein-containing gas prepared in the same manner as described in Example was allowed to pass through a fixed-bed oxidation reactor (the fixed bed thereof was composed of the catalyst for producing acrylic acid) in the same manner as described in Example. The gas flowing out of the reactor after 24 hours or later from the start of the passing was absorbed in water to obtain an aqueous acrylic acid solution. This aqueous acrylic acid solution was supplied to a solvent separation tower, and low boiling point impurities such as water and acetic acid were removed from the aqueous acrylic acid solution by azeotropic distillation. Then, the aqueous acrylic acid solution was supplied to the tower bottom of a high boiling point impurity separation tower having fifty weir-free porous plates and distilled while setting the reflux ratio to be 2. At the time of the distillation in this high boiling point impurity separation tower, p-methoxyphenol was charged from the top of the separation tower and hydrazine hydrate was charged on the 25-th weir-free porous plate from the bottom of the separation tower, to thereby obtain an acrylic acid-containing composition containing acrylic acid and 20 ppm by mass of p-methoxyphenol from the top of the separation tower.

Then, 72.07 g of the above acrylic acid-containing composition, 293.06 g of ion exchanged water, and polyethylene glycol diacrylate (the average addition mole number of ethylene oxide thereof was 8.2) in a concentration of 0.05 mol% relative to the total amount of monomers were mixed to prepare an aqueous monomer solution having an acrylic acid concentration of 20 mol%, a p-methoxyphenol concentration of 4 ppm by mass, and a neutralization rate of 0 mol%.

The total amount of the aqueous monomer solution at a temperature of 20°C, which had been prepared as described above, was charged in a vessel for polymerization (a cylindrical vessel made of polypropylene, having a capacity of 1 L), into which nitrogen gas was blown to allow the concentration of dissolved oxygen to be 1 ppm or lower in the aqueous monomer solution. Then, the vessel for polymerization was kept in an adiabatic state, to which an aqueous solution containing 0.12 g of sodium persulfate as a polymerization initiator and an aqueous solution containing 0.0018 g of L-ascorbic acid were added. Then, the polymerization of acrylic acid was promoted until it passed 30 minutes from the time when the temperature of the aqueous monomer solution became a peak temperature, to thereby obtain a water-containing gel polymer. The water-containing gel polymer was made minute to a size of about 1 mm, to which 62.5 g of a 48 wt% aqueous sodium hydroxide solution was added to neutralize 75 mol% of the acid groups of the water-containing gel polymer. After the neutralization, the calculated polymerization ratio of the water-containing gel polymer was 98.4%. Then, the water-containing gel polymer was spread over a net having a mesh of 850 µm and dried in hot blow of a gas (having a dew point of 160°C) at 60°C for 60 minutes, crushed by a vibration mill, and further classified with a JIS standard sieve having a mesh of 850 µm. The powder passing the sieve in this classification was obtained as a water-absorbing resin.

### Physical properties of water-absorbing resin

The physical properties of the water-absorbing resin were 50 times as the absorption factor to physiological saline, 48 times as the absorption factor to artificial urine, and 9% by mass of water-soluble components. In this regard, the measurement methods of the respective physical properties of the water-absorbing resin were as follows.

### Absorption factor to physiological saline

According to the absorption factor calculation method under no load conditions as disclosed in U.S. Patent No. 5,164,459, the absorption factor was calculated as follows. First, 0.2 g of the water-absorbing resin was charged in a tea bag type non-woven fabric bag (having a size of 40 mm x 150 mm), and then, the opening portion of the non-woven fabric bag was closed and sealed. The non-woven fabric bag was immersed in 100 g of physiological saline (i.e., a 0.9 wt% aqueous sodium chloride solution) at a temperature of 25 ± 3°C for 30 minutes. After the immersion, draining was carried out. The absorption factor ((W1 - W2) g / 0.2 g) of the water-absorbing resin to the physiological saline was calculated from the mass (W2) of the water-absorbing resin and non-woven fabric bag before immersion in the physiological saline and the mass (W1) of the water-absorbing resin and non-woven fabric bag after immersion and draining measured, respectively, in this series of operations.

### Absorption factor to artificial urine

According to the absorption factor calculation method under no load conditions as disclosed in U.S. Patent No. 5,164,459, the absorption factor was calculated as follows. First, 0.2 g of the water-absorbing resin was charged in a tea bag type non-woven fabric bag (having a size of 60 mm x 60 mm), and then, the opening portion of the non-woven fabric bag was closed and sealed. The non-woven fabric bag was immersed in 100 g of artificial urine at a temperature of 25 ± 3°C for 60 minutes. After the immersion, draining was carried out at 250 G for 3 minutes by using a centrifuge machine. The absorption factor ((W3 - W4) g /0.2 g) of the water-absorbing resin to the artificial urine was calculated from the mass (W4) of the water-absorbing resin and non-woven fabric bag before immersion in the artificial urine and the mass (W3) of the water-absorbing resin and non-woven fabric bag after immersion and draining measured, respectively, in this series of operations. In this regard, the artificial urine used herein was artificial urine commercially available by Jayco Inc. (i.e., a solution obtained by dissolving 2.0 g of potassium chloride, 2.0 g of sodium sulfate, 0.85 g of ammonium dihydrogenphosphate, 0.15 g of ammonium monohydrogenphosphate, 0.19 g of calcium chloride, and 0.23 g of magnesium chloride in 1 L of distilled water).

### Water-soluble components

First, 500 mg of the water-absorbing resin was dispersed in 1,000 ml of deionized water at room temperature, and the mixture was stirred by a magnetic stirrer for 16 hours. Then, the water-absorbing resin in the form of a swollen gel was filtered with a paper filter (No. 6, available from Toyo Roshi Kaisha., Ltd.). Then, the water-absorbing resin in the filtrate was subjected to colloid titration to determine the water-soluble components in the water-absorbing resin.

## Claims

1. A process for producing acrylic acid, comprising a refinement step of removing phenol and/or 1-hydroxyacetone from an acrolein-containing composition and an oxidation step of oxidizing acrolein in the acrolein-containing composition after the refinement step to produce acrylic acid.

2. The process for producing acrylic acid according to claim 1, further comprising a dehydration step of dehydrating glycerin to produce acrolein before the refinement step.

3. The process for producing acrylic acid according to claim 2, wherein glycerin is dehydrated in gas phase in the dehydration step.

4. A process for producing an acrylic acid derivative, comprising a step of producing acrylic acid by use of the production process according to any one of claims 1 to 3.

5. The process for producing an acrylic acid derivative according to claim 4, wherein the acrylic acid derivative is a water-absorbing resin.

6. An apparatus for producing acrylic acid, comprising a refiner for removing phenol and/or 1-hydroxyacetone from an acrolein-containing composition and an oxidation reactor for oxidizing acrolein in the acrolein-containing composition refined by the refiner to produce acrylic acid.

7. A composition for producing acrylic acid, comprising acrolein, wherein a ratio (P/A) of a mass (A) of acrolein and a mass (P) of phenol is 0.020 or lower and a ratio (H/A) of a mass (A) of acrolein and a mass (H) of 1-hydroxyacetone is 0.020 or lower.
